(19) Europäisches Patentamt
European Patent Office
Office européen des brevets

(11) Publication number: **0 256 805 A2**

(12) **EUROPEAN PATENT APPLICATION**

(21) Application number: **87307012.2**

(22) Date of filing: **07.08.87**

(51) Int. Cl.⁴: **A 61 K 31/445**
A 61 K 31/415, A 61 K 31/44
//(A61K31/445,31:415),
(A61K31/445,31:44)

(30) Priority: **08.08.86 GB 8619450**

(43) Date of publication of application:
**24.02.88 Bulletin 88/08**

(84) Designated Contracting States:
**BE CH DE FR GB IT LI NL SE**

(71) Applicant: **GLAXO GROUP LIMITED**
**Clarges House 6-12 Clarges Street**
**London W1Y 8DH (GB)**

(72) Inventor: **Humphrey, Patrick Paul Anthony**
**New Sands End Harlton Road**
**Little Eversden Cambridgeshire (GB)**

**Lumley, Philip**
**7 St. John's**
**Puckeridge Hertfordshire SG11 1SY (GB)**

(74) Representative: **Skailes, Humphrey John et al**
**Frank B. Dehn & Co. Imperial House 15-19 Kingsway**
**London WC2B 6UZ (GB)**

(54) **Pharmaceutical compositions for the treatment of occlusive vascular diseases.**

(57) The use is described of both (i) [1R-[1α(Z),2β, 3β,5α]]-(+)-7-[5-[[(1,1'-biphenyl)-4-yl]methoxy]-3-hydroxy-2-(1-piperidinyl)cyclopentyl]-4-heptenoic acid or a physiologically acceptable salt or solvate thereof and (ii) a thromboxane synthase inhibitor, either separately or in combination in the therapy or prophylaxis of occlusive vascular diseases in humans.

Pharmaceutical compositons containing both (i) and (ii) are also described.

EP 0 256 805 A2

## Description

PHARMACEUTICAL COMPOSITIONS FOR THE TREATMENT OF OCCLUSIVE VASCULAR DISEASES

This invention relates to improvements in the treatment of occlusive vascular diseases, and in particular to improvements in anti-thrombotic therapy.

Thromboxane synthase inhibition has received much attention for its potential in the treatment of occlusive vascular diseases. However, a major problem following thromboxane synthese inhibition is an accumulation of prostaglandin endoperoxides which can occupy and activate the thromboxane receptors and therapy induce platelet aggregation and secretion. In contrast, thromboxane receptor antagonists, by blocking the thromboxane/endoperoxide receptor, prevent the action of both thromboxane $A_2$ and the endoperoxides on platelets and smooth muscle.

Our UK Patent Specification 2097397 describes inter alia [IR-[1α(Z),2β,3β,5α]]-(+)-7-[5-[[(1,1'-biphenyl)-4-yl]methoxy]-3-hydroxy-2-(1-piperidinyl)cyclopentyl]-4-heptenoic acid (hereinafter referred to as Compound A). The compound is a potent thromboxane receptor blocker and thus antagonises the actions of thromboxane $A_2$, and, in particular, it inhibits thromboxane $A_2$ and endoperoxide mediated aggregation of blood platelets and contraction of vascular smooth muscle. It is thus of particular interest as an anti-thrombotic agent.

The present invention is based on our discovery that Compound A or a salt thereof (e.g. the hydrochloric salt) will act synergistically with a thromboxane synthase inhibitor to prevent blood platelet aggregation. In our experiments, using the test described by Lumley & Humphrey (J. Pharmacol. Methods, 1981, 6, 153-166) with collagen as the pro-aggregatory agent, we have shown that Compound A or a salt thereof (e.g. the hydrochloric salt) and a thromboxane synthase inhibitor such as 4-[2-(1H-imidazol- 1-yl)ethoxy]benzoic acid (dazoxiben) or (E)-7-phenyl-7-(3-pyridinyl)-6-heptenoic acid (CV 4151) can each inhibit platelet aggregation. However, when we have tested Compound A or a salt thereof (e.g. the hydrochlorid salt) in the presence of thromboxane synthase inhibitors such as dazoxiben or CV 4151 in isolated human whole blood, we have found that the effectiveness of the combination in inhibiting collagen-induced aggregation is greater than with either agent alone and is greater than any simple additive effect one might expect when using two anti-aggregatory agents which interfere with the actions of thromboxane.

Thus, the use of Compound A or a salt thereof (e.g. the hydrochlorid salt) with a thromboxane synthase inhibitor in combination therapy can be expected to be significantly more effective in the treatment of occlusive vascular diseases than is a thromboxane $A_2$ antagonist or a thromboxane synthase inhibitor alone.

Compound A or a salt thereof (e.g. the hydrochloric salt) and a thromboxane synthase inhibitor in combination therapy is therefore of interest for using in the treatment or prophylaxis of occlusive vascular diseases, including myocardial infarction, cardiac fatalities, angina, transient ischaemic attacks and cerebral infarction, atherosclerosis and vessel wall disease, peripheral vascular disease, diabetic nephropathy and retinopathy, postoperative thrombosis and pulmonary embolism, renal dialysis and peptic ulcer disease, peri- and postoperative complications following organ transplantation (particularly cardiac and renal), coronary artery bypass, angioplasty, thrombolysis and endarterectomy. Compound A or a salt thereof (e.g. the hydrochloride salt) and a thromboxane synthase inhibitor in combination therapy may also be of use in the treatment of asthma.

According to one aspect of the invention we therefore provide the use of a composition containing as active ingredients Compound A or a physiologically acceptable salt or solvate thereof and a thromboxane synthase inhibitor in the therapy or prophylaxis of occlusive vascular diseases in human subjects.

In a further aspect of the invention, we provide a method of treating occlusive vascular diseases in human subjects which comprises administering to the patient effective amounts of Compound A or a physiologically acceptable salt or solvate thereof and a thromboxane synthase inhibitor in a singe composition.

It will be appreciated that it is not necessary to administer Compound A or a salt or solvate thereof and the thromboxane synthase inhibitor as a single composition in order to achieve an improvement in efficacy. Providing that both compounds are present at the same time in the subject to be treated the compounds may be administered separately, Compound A or a salt or solvate thereof preferably being administered first, followed by the thromboxane synthase inhibitor.

In another aspect of the invention, therefore, we provide the use of a Compound A or a physiologically acceptable salt or solvate thereof and a thromboxane synthase inhibitor in the presence of each other in the patient, for the therapy or prophylaxis of occlusive vascular diseases in human subjects.

In a further aspect of the invention we provide a method of treating occlusive vascular diseases in human subjects which comprises administering to the patient effective amounts of Compound A or a physiologically acceptable salt or solvate thereof and a thromboxane synthase inhibitor in two separate compositions.

According to another aspect of the present invention we provide a pharmaceutical composition containing as active ingredients Compound A or a physiologically acceptable salt or solvate (e.g. hydrate) thereof, and a thromboxane synthase inhibitor.

Suitable salts of Compound A for use in the compositions of the invention include acid addition salts derived from inorganic and organic acids, such as hydrochlorides, hydrobromides, sulphates, phosphates, maleates, tartrates, citrates, benzoates, 2-chlorobenzoates, p-toluenesulphonates, methanesulphonates, salicylates, fumarates, lactates, hydroxynaphthalenecarboxylates (e.g. 1-hydroxy- or 3-hydroxy-2-naphthalenecarboxylates) or furoates; or salts with suitable bases such as alkali metal (e.g. sodium and potassium), alkaline earth metal (e.g. calcium or magnesium), ammonium and substituted ammonium (e.g. dimethylammonium, triethylammonium, 2-hydroxyethyl dimethylammonium, piperazine, N,N-dimethylpiperazine, piperidine, ethylenediamine and choline) salts. A preferred salt of Compound A for use in the composition according to the invention is the hydrochloride salt which is specifically described in UK Patent Specification 2127406.

Compound A may be prepared according to the methods described in UK Patent Specification 2097397.

The thromboxane synthase inhibitors for use in the compositions according to the invention may, in general, be any thromboxane synthase inhibitors which act in a synergistic manner with Compound A to effect inhibition of blood platelet aggregation. Suitable compounds may be determined empirically by testing a combination of each of the compounds with Compound A using the method of Lumley & Humphrey described herein.

Examples of known compounds which may be used with Compound A in compositions according to the invention includes imidazoles, imidazopyridines and pyridines with thromboxane synthase inhibitory activity. Particular examples of imidazole compounds include those described in UK Patent Specifications 2016452, 2025946, 2038821 and 2045244, European Patent Specifications 3901 and 33432 and Japanese Patent Specification J61277670. Particular examples of imidazopyridine compounds include those described in UK Patent Specification 2101595. Particular examples of suitable pyridine compounds include those described in UK Patent Specification 2039903 and European Patent Specifications 69521, 80154, 111997 and 129051.

Important thromboxane synthase inhibitors for use in the compositions of the invention include the following compounds of formula (1)

(a) 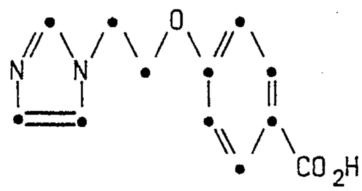 (dazoxiben)

(b) 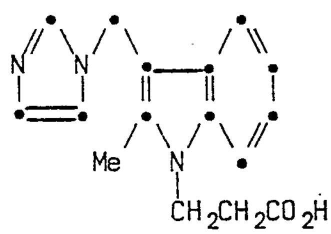 (dazmegrel)

(c) 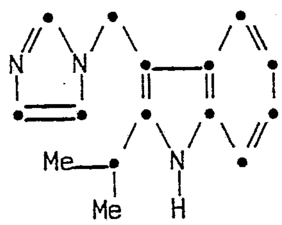 (UK 34787)

(d) 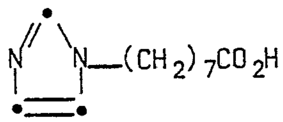

(e) 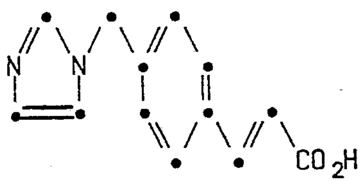 (OKY 046)

(f) 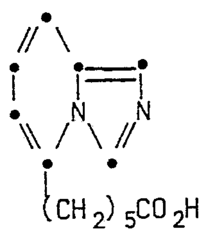 (CGS 13080)

(g) 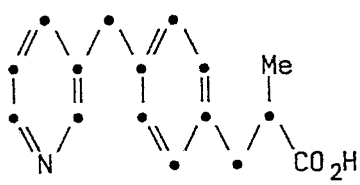 (OKY 1580)

(h) 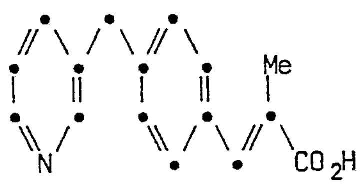 (OKY 1555)

(i) 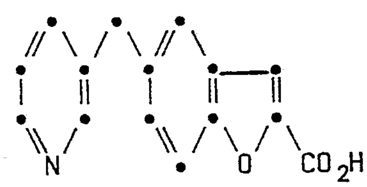 (furegrelate)

(j) 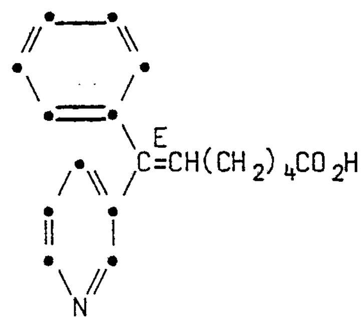 (CV 4151)

(k) 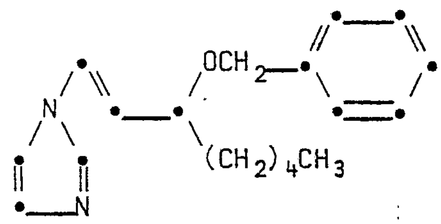 (midazogrel)

(ℓ) 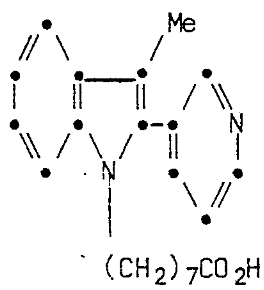 (CGS 12970)

(m) 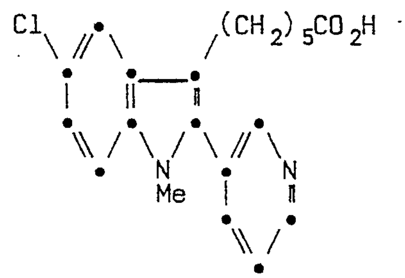 (CGS 15435)

and (n) 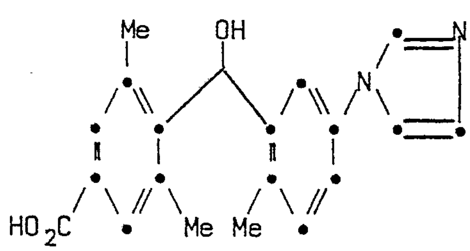 (Y 20811)

or a physiologically acceptable salt thereof.

Expecially important compounds are the compounds of formulae 1(a), 1(b) and 1(j) and, in particular, 1(j),

and the physiologically acceptable salts thereof.

Each of the compounds of formula (1) is described in one of the aforementioned UK, European and Japanese patent specifications and may be prepared by the methods described therein.

The relative proportions of Compound A and thromboxane synthase inhibitor employed in the compositions of the invention may generally be, for example, in the ratios by weight of about 25:1 to 1:10, in particular about 10:1 to 1:5 and especially about 1:1 of Compound A to synthase inhibitor.

The amound of Compound A employed in the compositions of the invention will preferably be in the range of 3.5 to 350mg particularly 3.5 to 100mg per dosage unit.

The amount of thromboxane synthase inhibitor employed in the compositions of the invention will preferably be in the range of 5 to 500mg per dosage unit.

It is to be understood that when Compound A and the thromboxane synthase inhibitor are administered in two separate compositions the amount of Compound A employed in one composition and the amount thromboxane synthase inhibitor employed in the other composition will preferably be in the ranges given just above.

The precise dose administered of both Compound A and the thromboxane synthase inhibitor will of course depend on the age and condition of the patient. In general, the composition(s) will be administered in 1-4 doses per day.

According to a further aspect of the invention we provide a pharmaceutical composition containing as active ingredients Compound A or a physiologically acceptable salt or solvate (e.g. hydrate) thereof, and a thromboxane synthase inhibitor for use in the therapy or prophylaxis of occlusive vascular diseases in human subjects.

The compositions of the invention may be prepared by admixture of the active ingredients, and according to a further aspect of the present invention we provide a process for the preparation of a pharmaceutical composition comprising admixing Compound A or a physiologically acceptable salt or solvate thereof and a thromboxane synthase inhibitor.

The compositions of the invention may be presented with the acid of at least one pharmaceutical carrier or excipient. Thus, in a further aspect of the invention we provide a pharmaceutical composition comprising as active ingredients Compound A or a physiologically acceptable salt or solvate thereof and a thromboxane synthase inhibitor together with one or more pharmaceutical carriers or excipients.

In a still further aspect of the invention we provide a process for the preparation of a pharmaceutical composition which comprises admixing Compound A or a physiologically acceptable salt or solvate thereof and a thromboxane synthase inhibitor together with one or more pharmaceutical carriers or excipients.

The compositions may be in any forms suitable for administration, particularly for oral or parenteral administration.

The compositions may take the form of, for example, tablets, capsules, powders, solutions or syrups for oral administration. The compositions may thus contain as excipients, for example, binding agents, compression aids, fillers, lubricants, disintegrants and wetting agents. Tablets may be coated in a conventional manner, for example with a suitable film-forming material such as methyl cellulose or hydroxypropylmethyl cellulose. Alternatively the tablets may be sugar coated. Liquid preparations may also contain, for example, edible oils such as peanut oil.

For parenteral administration the compositions of the invention may take a form suitable for continuous infusion. Such forms include suspensions, solutions or emulsions in oil or aqueous vehicles, which may optionally contain formulatory agents such as suspending, stabilising and/or dispersing agents. Alternatively, the active ingredients may be in powder form for reconstitution before use with a suitable vehicle, e.g. sterile pyrogen-free water. Formulations for injections may be presented in unit dosage form in ampoules, or in multi-dose containers, with an added preservative, or in a suitable container for infusion.

The compositions of the invention may be prepared according to methods well known in the pharmaceutical industry. Thus, for example, tablets may be prepared by direct compression of the active ingredients blended with appropriate excipients. Alternatively, the blend of active ingredients and excipients may first be granulated using conventional techniques and the resulting granules compressed into tablets. Tablets may be film coated with suitable film forming materials using standard techniques.

Capsules may be prepared by blending the active ingredients and excipients and then filling the blend into gelatin capsules using a suitable filling machine.

Solutions for parenteral administration may be prepared by dissolving the active ingredients in a suitable vehicle e.g. water, and adjusting the tonicity and pH of the solution required. The solution may if desired be clarified and then filled into appropriate sized ampoules. Sterilisation may be carried either before or after filling. If desired the solution may be packed under an inert atmosphere of nitrogen.

According to a still further aspecto of the present invention we provide a composition containing as active ingredients Compound A or a physiologically acceptable salt or solvate thereof and a thromboxane synthase inhibitor for use in the manufacture of a medicament for the therapy or prophylaxis of occulasive vascular diseases in human subjects.

It may be convenient to present Compound A and the thromboxane synthase inhibitor as a two container pack, one container containing Compound A and the other containing the thromboxane synthase inhibitor. The compounds may then be admixed immediately before administration, or, if desired, may be administered sequentially.

The invention also provides Compound A or a physiologically acceptable salt or solvate thereof and a thromboxane synthase inhibitor and compositions containing them, in association with instructions for their use together in the therapy or prophylaxis or occlusive vascular diseases in human subjects.

When Compound A or a physiologically acceptable salt or solvate thereof and the thromboxane synthase inhibitor are administered in two separated compositions such compositions may be prepared according to methods described in the aforementioned UK, European and Japanese patent specifications.

The following Examples illustrate pharmaceutical compositions according to the invention. In the Examples the term 'Thromboxane $A_2$ Antagonist' means Compound A or a physiologically acceptable salt thereof, especially the hydrochloric salt. The term 'Thromboxane Synthase Inhibitor' means, for example, a compound of formula (1) or a physiologically acceptable salt thereof and is especially a compound of formula 1(a), 1(b), or 1(j) and, in particular 1(j) or a physiologically acceptable salt thereof.

TABLETS

| | mg/tablet |
|---|---|
| Thromboxane $A_2$ Antagonist | 20.0 |
| Thromboxane Synthase Inhibitor | 20.0 |
| Microcrystalline cellulose | 159.0 |
| Magnesium stearate BP | 1.0 |
| Compression weight | 200.0mg |

The thromboxane $A_2$ antagonist and thromboxane synthase inhibitor are sieved and blended with the microcrystalline cellulose and magnesium stearate. The resulting mix is compressed on a suitable tablet machine using 8mm punches. Tablets of other strengths may be prepared by altering the ratio of the active ingredients to microcrystalline cellulose or the compression weight and using punches to suit.

The tablets may be film coated with suitable film forming materials, such as hydroxypropyl methylcellulose, using standard techniques. Alternatively the tablets may be sugar coated.

Tablets may also be prepared by other conventional methods such as wet granulation.

CAPSULES

| | mg/capsule |
|---|---|
| Thromboxane $A_2$ Antagonist | 20.0 |
| Thromboxane Synthase Inhibitor | 20.0 |
| Starch 1500* | 159.0 |
| Magnesium stearate BP | 1.0 |
| Fill Weight | 200.0mg |

* a form of directly compressible starch

The thromboxane $A_2$ antagonist and thromboxane synthase inhibitor are sieved and blended with the Starch 1500 and the excipients. The mix is filled into size No. 2 hard gelatin capsules using suitable machinery. Capsules of other strengths may be prepared by altering the ratio of the active ingredients to Starch 1500 or the fill weight and if necessary changing the capsule size to suit.

## Claims

1. The use of both (i) [1R-[1α(Z),2β,3β,5α]]-(+)-7-[5-[[(1,1′-biphenyl)-4-yl]methoxy]-3-hydroxy-2-(1-piperidinyl)cyclopentyl]-4-heptenoic acid or a physiologically acceptable salt or solvate thereof and (ii) a thromboxane synthase inhibitor either separately or in combination in the manufacture of medicaments for use in the therapy or prophylaxis of occlusive vascular diseases in humans.

2. The use according to claim 1 in which the compounds (i) and (ii) are presented as separate compositions for said use.

3. The use of both compounds (i) and (ii) as defined in claim 1, either separately or in combination, in the therapy or prophylaxis of occlusive vascular diseases in humans.

4. A pharmaceutical composition which comprises both a compound (i) and a compound (ii) as defined in claim 1.

5. The use or a composition according to any preceding claim, in which compound (i) is in the form of its hydrochloride salt.

6. The use or a composition according to any preceding claim, in which compound (ii) is dazoxiben or dazmegrel or a physiologically acceptable salt thereof.

7. The use or a composition according to any preceding claim in which compound (ii) is CV 4151 or a physiologically acceptable salt thereof.

8. The use or a composition according to any preceding claim, in which the ratio of compound (i) to compound (ii) is about 1:1 by weight.

9. The use or a composition according to any preceding claim, in which compounds (i) and (ii) are in a form suitable for oral or parenteral administration.

10. The use or a composition according to claim 9, in which compounds (i) and (ii) are in the form of tablets, capsules or ampoules containing unit doses thereof.

11. A two-container pack for use in the therapy or prophylaxis of occlusive vascular diseases in humans, one of the containers containing (i) and the other containing (ii) as defined in claim 1.

12. A composition according to any of claims 4 to 10, or a pack according to claim 11 or (i) or (ii) as defined in claim 1 in association with instructions for the use of both (i) and (ii) in the therapy or prophylaxis of occlusive vascular diseases in humans.